# EUROPÄISCHE PATENTSCHRIFT

(11) **EP 4 424 282 B1**
(45) Veröffentlichungstag und Bekanntmachung des Hinweises auf die Patenterteilung: **13.08.2025**
(21) Anmeldenummer: 24160148.3
(22) Anmeldetag: 28.02.2024
(51) Int. Cl.: A61F 2/78, A61F 2/80

(54) **SCHICHTSYSTEM ZUR ANBRINGUNG UND/ODER ENTFERNUNG EINES KÖCHERS AN EINEN STUMPF**
LAYER SYSTEM FOR ATTACHING AND/OR REMOVING A CASING TO A STUMP
SYSTÈME DE COUCHES DESTINÉ À L'APPLICATION ET/OU À L'ÉLIMINATION D'UN CORPS SUR UN MOIGNON

(30) Priorität: 02.03.2023 DE 202023100985 U
(43) Veröffentlichungstag der Anmeldung: 04.09.2024
(73) Patentinhaber: Merbold, Daniel, 6345 Kössen (AT)
(72) Erfinder: Merbold, Daniel, 6345 Kössen (AT)
(74) Vertreter: Kailuweit & Uhlemann Patentanwälte Partnerschaft mbB

(56) Entgegenhaltungen:
- DE-A1- 10 237 269
- US-A1- 2020 060 846
- US-A1- 2022 296 394
- US-B2- 10 335 295

## Beschreibung

Die vorliegende Erfindung betrifft ein Schichtsystem zur Anbringung und/oder Entfernung eines Köchers an einen Stumpf. Weiterhin betrifft die Erfindung eine Prothese aufweisend das erfindungsgemäße Schichtsystem. US 10 335 295 B2 wird als nächstliegender Stand der Technik angesehen.

Nach einer Amputation von Gliedmaßen werden in der Regel Körperersatzstücke, im Weiteren auch als Prothese bezeichnet, angepasst, welche an den verbliebenen Amputationsstumpf angebracht werden. Als Amputationsstumpf bezeichnet man den proximal verbleibenden Rest einer amputierten Extremität. Im Weiteren wird der Amputationsstumpf auch als Stumpf, Körperstumpf oder Gliedmaßenstumpf bezeichnet. Distal beschreibt in der Medizin eine anatomische Lagebezeichnung und bedeutet "weiter vom Rumpf entfernt", "von der Körpermitte weg" oder "von einem Bezugspunkt (z.B. Gefäßursprung) entfernt gelegen". Das Gegenteil von "distal" ist "proximal".

Unter einer Prothese versteht man i.Allg. den künstlichen Ersatz eines fehlenden, amputierten oder unvollständig ausgebildeten Körperteils, besonders der Gliedmaßen oder der Zähne. Es werden Exoprothesen von Endoprothesen unterschieden. Unter Endoprothesen werden Körperstrukturen (z.B. Gelenke oder Blutgefäße) verstanden, die in der Regel dauerhaft im Körper verbleiben. Exoprothesen sind hingegen orthopädische Hilfsmittel, die als Körperersatzstücke dem funktionellen und ästhetisch-kosmetischen Ausgleich von amputierten oder von Geburt an fehlenden Gliedmaßenabschnitten dienen. Der Begriff Prothese wird in dieser Erfindung als Substitution für Gelenke bzw. andere Körperteile (insbesondere Beine und Arme aber nicht darauf beschränkt), die ihre Funktion nicht ausreichend wahrnehmen können verwendet.

Dabei umfasst der Begriff der Beinprothese Prothesen im Bereich des Unterschenkels und des Oberschenkels sowie Knieexartikulation-Prothesen. Ferner umfasst der Begriff der Armprothese Prothesen im Bereich des Unterarms oder Oberarms sowie Exartikulationsprothesen.

Eine Prothese kann sowohl bei Menschen als auch bei Tieren eingesetzt werden. Bei Verwendung im veterinärmedizinischen Kontext sind spezifische Begrifflichkeiten der Gliedmaßen entsprechend anzupassen.

Bestandteile herkömmlicher Prothesen umfassen Prothesenbauteile, einen Köcher und mindestens einen Liner. Die Prothesenbauteile können die Anatomie und Funktion dieses Körperteils wie beispielsweise eines Beines, aufweisen. Bei den Prothesenbauteilen handelt es sich ferner um kommerziell erhältliche Bestandteile, zu welchen u.a. auch alle zum Halt des anatomisch nachgebildeten Körperteils dienen, bspw. Absperrorgane, Stifte, Verschlüsse.

Die Prothesenbauteile sind in der Regel mit einem Köcher verbunden, welcher jeweils in Maßarbeit und anatomisch individuell von einem Fachhandwerker z.B. einem Orthopädietechniker angepasst und gefertigt wird. Der Köcher dient ferner als Anbindungsstelle zwischen Körper und Prothesenbauteil. Der Köcher nimmt hierzu einen Liner auf, wobei der Liner wiederum mit dem Körperstumpf in Kontakt ist.

Ein Liner ist eine Verbindung zwischen Stumpf und Prothese. Er wird als eng anliegendes Kleidungsstück (z.B. ähnlich eines Strumpfs) über den Stumpf gezogen. Durch die gleichmäßige Belastung und seine Form soll der Liner den Stumpf vor unangenehmen Druckstellen, Hitze und Feuchtigkeit schützen und für einen möglichst hohen Tragekomfort sorgen. Der Liner entspricht einem ringförmigen Schlauch, welcher an einem Ende- dem proximalen Ende - offen ausgebildet ist, um den Stumpf aufzunehmen und an seinem anderen Ende - dem distalen Ende - geschlossen ausgebildet ist. Der Liner ist über einen Haftungsmechanismus mit dem Köcher verbunden. Hierzu sind verschiedene Methoden bekannt, welche flexibel und individuell gewählt werden können. So kann die Haftung beispielsweise über einen im Liner angebrachten Stift gewährleistet werden, welcher in ein korrespondierendes Schloss am Köcherboden einrastet. Nachteilig wird dadurch ein Hub- bzw. Saugeffekt erzeugt, indem sich bei jeder Bewegung des Prothesenträgers der Liner vom Köcher lösen kann.

Eine zuverlässigere Methode, die Haftung zwischen Liner und Köcher herzustellen, ist die Verwendung von Vakuumsystemen. So kann ein passiv ausgebildetes Vakuum bzw. Unterdruck durch ein Einwegventil oder ein aktiv ausgebildetes Vakuum bzw. Unterdruck durch ein komplexeres Saugsystem erreicht werden.

Eine weitere zuverlässige Haftung wird durch das Überstülpen eines Strumpfes, eines Gurtes oder einer Kniekappe über den Liner erreicht, wodurch die Luftschicht zwischen Liner und Köcher extrahiert wird. Diese Methode geht allerdings mit der größten Einschränkung hinsichtlich der Bewegungsfreiheit einher.

Bei Stoffen und Kleidungsstücken wird in der Regel eine Außen- und Innenseite unterschieden. Die Außenseite hat dann die bei der Produktion gewünschte Optik bzw. Funktion, welche durch Veredelungsmaßnahmen oder durch Umschlag der Gewebekanten erreicht wird. Statt von einer Außen- und Innenseite spricht man in der Textilbranche allerdings von einer rechten und linken Seite. Die beiden Begriffe rechts und links sind in dem Fall keine Richtungsangaben, sondern stehen für die korrekte bzw. richtige Seite (rechts) und die Ab- bzw. Rückseite (links). Im Sinne der Erfindung ist mit linker Seite die stumpfzugewandte und mit rechter Seite die stumpabgewandte Seite gemeint. Durch den Liner und dessen Materialeigenschaften kann der Köcher hoch haftend und gepolstert am Körper angebunden werden. Da sie luftdicht auf der Haut sitzen müssen, sind Liner auf links gedreht und müssen so auf den Stumpf aufgerollt werden. Dies erfolgt, indem der auf links gedrehte, geschlossen ausgebildete Bereich des Liners auf das distale Ende des Stumpfes angelegt und ausgerollt wird. Der geschlossen ausgebildete Bereich umschließt den Stumpf im aufgerollten Zustand vollständig.

Für additive Fertigungsverfahren, Gieß- oder Walzverfahren eigenen sich insbesondere Silikonkautschuk RTV/NV, HTV-Silikon-Kautschuke oder UV-vernetzbares Silikon. Der Silikonkautschuk RTV/NV ist ein gut fließfähiger Silikonkautschuk mit niedriger Viskosität und mittlerer Elastizität. HTV-Silikon-Kautschuke sind plastisch verformbare Materialien. Sie enthalten häufig organische Peroxide für die Vernetzung. Die daraus durch die Vernetzung bei hoher Temperatur hergestellten Elastomere sind wärmebeständige.

### Stand der Technik

In der EP 3 171 826 A1 / WO002016012094A1 wird ein Liner für eine Prothese beschrieben. Der Liner weist eine Innenseite und eine Außenseite auf, zwischen denen mindestens ein Strömungskanal mit einem Einlass und Auslass verläuft, welcher ein Ventil zur Regulierung der Strömung aufweist. Weiterhin ist zwischen der Innenseite und der Außenseite wenigstens eine Pumpkammer in Fluidverbindung mit dem wenigstens einem Strömungskanal angeordnet. Dadurch wird ein Vakuum zwischen dem distalen Ende des Liners und dem Köcher generiert, indem die Bewegung des Stumpfes benutzt wird.

Die US 8 080 065 B2 offenbart eine in der Anfertigung aufwendig ausgebildete Vorrichtung zum Befestigen an einen Körperstumpf. Dazu wird ein Liner auf einen Stumpf aufgerollt, wobei der Liner weitere Linerschürzen aufweist, die konzentrisch um den Liner angeordnet sind und um diesen geklappt werden können, um eine Luftdurchdringungseinrichtung aufzunehmen. Diese Einrichtung realisiert einen zusätzlichen Sicherungsmechanismus für das Prothesenbauteil und verhindert Lufteinschlüsse zwischen Liner und einem daran befestigten Köcher. Durch die Luftdurchdringungseinrichtung wird ferner die Erzeugung eines Vakuums beim Einführen des Stumpfes in den Köcher erleichtert.

Die US 6 761 742 B2 beschreibt eine gewichtsbetätigte Vakuumpumpe für eine hypobarische kontrollierte künstliche Gliedmaße. In diesem Zusammenhang wird vorgeschlagen über einen Liner eine luftgängige Textillage aufzubringen, auf der dann der Prothesenschaft aufsitzt. Der Zwischenraum zwischen Liner und Prothesenschaft kann evakuiert werden. Eine Abdichtung erfolgt über eine zusätzliche Manschette, die sich vom unbedeckten Stumpf über das proximale Ende des Liners und Schaftes erstreckt. Aufgrund der textilen Lage wirkt der Unterdruck unmittelbar auf das Gewebe des Stumpfes.

Das Gebrauchsmuster DE 20 2007 015 828 U1 zeigt einen doppelwandig ausgebildeten Liner für Vakuumschäfte, wobei ein Innenliner in Kontakt mit dem Stumpf ist und ein Außenliner in den Köcher gesetzt wird. Dabei sind Innen- und Außenliner im mittleren bis distalen Drittel fest miteinander verbunden. Das für den Halt benötigte Vakuum wird durch eine zirkulär angeordnete Dichtlippe direkt zwischen dem Köcher und dem Liner ausgebildet. Nachdem der Stumpf mit aufgerolltem Liner in den Köcher eingeführt wurde, wird der Außenliner über den Köcherrandverlauf distal umgeschlagen und dichtet so auf der Außenseite des Köchers hinreichend ab, wodurch ein Vakuum zwischen Köcher und Liner über die gesamte Köcherinnenfläche realisiert wird. Auf eine Luftdurchdringungseinrichtung kann dabei jedoch verzichtet werden.

Durch die aus dem Stand der Technik bekannten Liner wird im distalen Ende zwischen den Linerschichten eine feste Verbindung ohne Kraftwirkung hergestellt. Die eigentlich zwischen Stumpf und Prothesenbauteil vorliegende Kraftwirkung erfolgt zwischen der äußeren Linerschicht und dem Köcher.

Aus dem Stand der Technik bekannten Liner können weit in den Proximalbereich des Körpers hineinragen. Zum Beispiel im Falle einer Unterschenkelprothese bis weit in den Oberschenkel oder zumindest so weit, dass die Kniekehle noch bedeckt ist. Dies führt häufig zu unerwünschten Hautirritationen, Haarwurzelentzündungen und Blasenbildung einhergehend mit Eigenschweißallergien an den vom Liner bedeckten Hautstellen, welche im Falle einer Unterschenkelprothese häufig durch die starken Bewegungen im Bereich des Kniegelenks und die üblicherweise vorhandenen konkaven und konvexen Formen rund um das Knie hervorgerufen werden.

Zwar können die Liner, um diese unerwünschten Effekte zu verhindern, entsprechend gekürzt werden, allerdings bringt dies weitere Probleme insbesondere an den Schnitträndern mit sich. Diese Bereiche sind besonders vulnerabler, da es hier vermehrt zu Spannungsblasen kommen kann. Spannungsblasen entstehen an Grenzgebieten, wo die Haut sich nicht ungehindert stauchen und dehnen kann, wie es bei der Ummantelung durch einen Liner der Fall ist.

Ein weiterer Nachteil von Schnittkanten bei Silikon-Linern, ist ein frühzeitiger Verschleiß an nicht versiegelten Rändern, da sich die äußere Stoffbeschichtung hier ausfransen und lösen kann.

Durch Geometrie und Bauweise bekannter Liner wird zudem nachteilig die Bewegungsfreiheit des Prothesenträgers eingeschränkt.

Bei Kopplung eines Liners mit distaler Arretierung unter Zuhilfenahme eines Verbindungselements (z.B. eines Stiftes) zur Verbindung einer Prothese treten immer Hub- und Saugeffekte auf, die störend wirken.

Aufgabe der Erfindung ist es, ein System bereitzustellen, welches die Nachteile des Standes der Technik überwindet. Dieses sind u.a. Verwendung von zusätzlichen Linerschürzen, Erzeugung eines notwendigen Vakuums, Vermeidung von Hub- und Saugeffekte oder eine aufwendige Lineranpassung.

Dabei soll ein effizient funktionierendes und einfach aufgebautes System zur Verfügung gestellt werden, welches Komfort und Bewegungsfreiheit für den Prothesenträger ermöglicht und längere Tragzeiten erlaubt.

Erfindungsgemäß wird die Aufgabe durch ein Schichtsystem zur Anbringung und/oder Entfernung eines Köchers an einen Stumpf gemäß dem unabhängigen Anspruch gelöst. Vorteilhafte Ausgestaltungen der Erfindung sind in den abhängigen Ansprüchen angegeben.

Dabei ist das erfindungsgemäße Schichtsystem derart ausgeführt, um durch Auf- bzw. Abrollen an einem Stumpf angebracht zu werden. Das erfindungsgemäße Schichtsystem stellt vorteilhaft einen innig ausgebildeten Kontakt zum Köcher her.

Im Gegensatz zu herkömmlichen Prothesen handelt es sich bei der vorliegenden Erfindung durch den Einsatz des erfindungsgemäßen Schichtsystems um voneinander getrennte Kraftsysteme, welche durch das Anbringen oder Entfernen der Prothese vorteilhaft nacheinander und nicht parallel geschaltet sind. So wird beim Anbringen zuerst die Adhäsionskraft zwischen Stumpf und Adhäsionsschicht durch die Eigenschaften des Materials der Adhäsionsschicht per se hergestellt, anschließend ein Kraft- und/oder Formschluss zwischen Adhäsions- und Kontaktschicht durch die Evakuierung mittels eines Absperrorgans erreicht und daraufhin eine weitere mechanische Verbindung durch Klemmkraft und/oder die Ausbildung eines Vakuums bzw. Unterdrucks und somit ein weiterer Kraft- und/oder Formschluss zwischen Kontaktschicht und Köcher erreicht. Die generierten Kräfte werden beim Entfernen der Prothese in umgekehrter Reihenfolge wieder abgebaut.

Ein erster Aspekt der Erfindung betrifft ein Schichtsystem zur Anbringung und/oder Entfernung eines Köchers an einen Stumpf. Im Sinne der Erfindung weist das Schichtsystem ein proximales und ein distales Ende auf und umfasst als Komponenten eine Adhäsionsschicht und eine Kontaktschicht. Das proximale bzw. distale Ende des Schichtsystems wird im Sinne der Erfindung auch als proximaler bzw. distaler Bereich bezeichnet. Im Fall einer Unterschenkelprothese entspricht das proximale Ende des Schichtsystems dem knienahen Bereich und das distale Ende ist jener Bereich, welcher sich auf Höhe des Stumpfendes befindet und somit in Köchernähe ist.

Erfindungsgemäß weist dabei die Adhäsionsschicht zwei Flächen auf, wobei die stumpfzugewandte Fläche der Adhäsionsschicht den Stumpf zumindest teilweise bedeckt und einen haftenden Kontakt zum Stumpf durch Ausbildung einer Adhäsionskraft herstellt. Dabei erfolgt die Adhäsionshaftung über molekulare Wechselwirkung in den Grenzflächen und bewirkt damit einen mechanischen Zusammenhalt der beteiligten Flächen. In Ausführungsformen ist der dabei entstehende Kontakt groß- bzw. vollflächig ausgebildet, sodass vorteilhaft die sich in Benutzung ausbildende haftende Adhäsionskraft zwischen Adhäsionsschicht, insbesondere der stumpfzugewandten Flächen der Adhäsionsschicht und Stumpf im Bereich einer groß ausgebildeten Fläche beim Tragen der Prothese gut wirken kann.

In weiteren Ausführungsformen ist der dabei entstehende Kontakt entlang der kompletten Berührungsfläche zwischen Adhäsionsschicht und Stumpf ausgebildet.

Durch das luftdichte Anliegen auf der Haut reichen die Adhäsionskräfte aus, einen stabilen Halt zu geben, es ist kein zusätzliches Vakuum bzw. Unterdruck nötig. In Ausführungsformen ist die Adhäsionsschicht als Adhäsionsstrumpf ausgebildet. Weiterhin erfindungsgemäß weist die Kontaktschicht zwei Flächen auf, wobei die Kontaktschicht über der stumpfabgewandten Fläche der Adhäsionsschicht angeordnet ist, indem die stumpfzugewandte Fläche der Kontaktschicht zumindest teilweise die stumpfabgewandte Fläche der Adhäsionsschicht bedeckt, wobei bei dieser Bedeckung eine möglichst geringe Adhäsion realisiert wird.

Im Sinne der Erfindung ist das Wort bedecken synonym zu verwenden mit umschließen. Dabei liegt die Kontaktschicht über der Adhäsionsschicht. Dabei entsprechen die stumpfzugewandten Flächen von Adhäsions- und Kontaktschicht jeweils den Innenflächen dieser Schichten, welche in Richtung Stumpf weisen, wobei die stumpfzugewandte Fläche der Adhäsionsschicht mit diesem zumindest teilweise in Kontakt ist. Die stumpfabgewandte Fläche von Adhäsions- und Kontaktschicht entsprechen jeweils den Außenflächen dieser Schichten, welche in Richtung Köcher weisen, wobei die stumpfabgewandte Fläche der Kontaktschicht mit diesem zumindest teilweise in Kontakt ist. Jene Flächen, wo sich die stumpfabgewandte Fläche der Adhäsionsschicht und die stumpfzugewandte Fläche der Kontaktschicht einander berühren, werden im Sinne der Erfindung als Kontaktfläche bezeichnet.

Durch den Einsatz einer Adhäsions- und einer Kontaktschicht werden vorteilhaft die Hub- und Saugeffekte überwunden. Weiterhin vorteilhaft kann durch den Einsatz der beiden Schichten zudem ein Verbindungselement eingesetzt werden, was für eine zusätzliche und effektive Sicherung sowie Halt sorgt. Insbesondere die Verwendung eines Stiftes als Verbindungselement, welcher für ein einfaches und schnelles Anbringen und Entfernen der Prothese dient, ist in der vorliegenden Erfindung möglich, ohne einen Hub- und Saugeffekt wie bei herkömmlichen Prothesen zu generieren.

Zudem vorteilhaft ist, dass der Einsatz eines zusätzlichen Sicherungsmechanismus, wie es bspw. in der US 8,080,065 B2 beschrieben wird, entfällt.

Im Sinne der Erfindung ist weiterhin die Kontaktschicht derart ausgebildet, um über deren stumpfabgewandten Fläche einen formschlüssigen Kontakt zum Köcher zu gewährleisten. Der sich dabei ausbildende formschlüssige Kontakt zwischen Kontaktschicht und Köcher wird durch die mechanische Verbindung aufgrund der Ausbildung einer Klemmkraft und/oder Vakuums bzw. Unterdrucks erreicht.

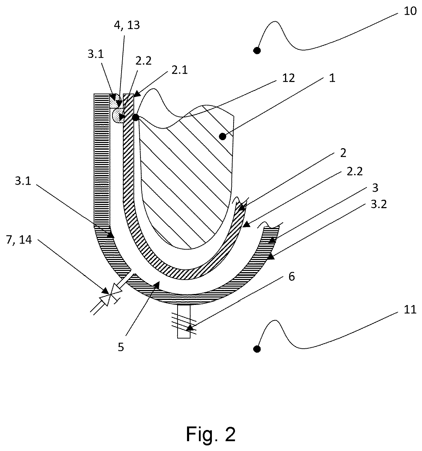

In weiteren Ausführungsformen ist der Kontakt kraftschlüssig oder kraft- und/oder formschlüssig ausgebildet. Dazu weist die Kontaktschicht ein Absperrorgan zur Evakuierung und/oder Belüftung des zwischen Adhäsionsschicht und Kontaktschicht gebildeten Hohlraumes und am distalen Ende ein Verbindungselement zur lösbaren Befestigung des Schichtsystems an den Köcher auf, wobei die Kontaktfläche zwischen der zweiten Fläche der Adhäsionsschicht und der ersten Fläche der Kontaktschicht am proximalen Ende formschlüssig und luftdicht ausgebildet ist (vgl. Fig. 2).

In weiteren Ausführungsformen ist die Kontaktfläche kraftschlüssig oder kraft- und/oder formschlüssig ausgebildet.

In weiteren Ausführungsformen kann durch die Haltevorrichtung zusätzlich ein Vakuum bzw. Unterdruck zwischen Kontaktschicht und Köcher gewährleistet werden. Zwischen der Adhäsions- und der Kontaktschicht entsteht dabei ein Kraft- und/oder Formschluss, welcher aufgrund des Verbindungselementes als Vakuum bzw. Unterdruck ausgebildet ist.

In Ausführungsformen ist die Kontaktschicht als Kontaktstrumpf ausgebildet. In Ausführungsformen weisen die Adhäsions- und die Kontaktschicht die gleiche Fläche auf bzw. sind diese deckungsgleich übereinander angeordnet.

In alternativen Ausführungsformen - sofern der Kraft- und/oder Formschluss und das Vakuum bzw. Unterdruck noch realisiert werden können - weist die Kontaktschicht eine kleinere Fläche als die Adhäsionsschicht auf und bedeckt diese insbesondere nur am proximalen Ende.

In Ausführungsformen, aber nicht darauf beschränkt, da abhängig von der Wahl des Materials, weisen die Adhäsions- und/oder Kontaktschicht eine Schichtdicke im Bereich von 2mm bis 6mm auf.

Im Sinne der Erfindung ist der Begriff Prothese als Exoprothese zu verstehen.

In einer bevorzugten Ausführungsform ist ein Absperrorgan am distalen Ende des Schichtsystems angeordnet. Vorteilhaft sorgt das Absperrorgan für das Ein- und Ausströmen der Luft in den Hohlraum zwischen Adhäsions- und Kontaktschicht.

In Ausführungsformen handelt es sich bei dem Absperrorgan um ein kommerzielles Ausstoßventil oder vergleichbares.

In einer bevorzugten Ausführungsform ist das Absperrorgan und das Verbindungselement als integriertes Bauteil ausgebildet. Dies bedeutet, dass das Absperrorgan die Funktion des Verbindungselements übernehmen kann und umgekehrt. Vorteilhaft kann dadurch ein Bauelement eingespart werden. In Ausgestaltungsformen handelt es sich um einen Magnetverschluss mit integriertem Ventil.

In einer bevorzugten Ausführungsform ist das Verbindungselement als Stift und/oder Verschlusssystem und/oder Absperrorgan und/oder Dichtungselement ausgebildet. Durch das Verbindungselement kann der Köcher vorteilhaft luftdicht an die Kontaktschicht gekoppelt werden.

In Ausführungsformen geht das Verbindungselement mit korrespondierenden Aufnahmen, welche sich an entsprechend für die Wahl des Verbindungselementes geeigneten Positionen am Köcher befinden, einen Formschluss ein. Dies betrifft insbesondere jene Ausgestaltungen des Verbindungselementes als Verschlusssystem bzw. Stift, sodass das Verbindungselement mit einem Gegenstück bzw. Schloss am Köcher den Formschluss realisiert. Vorteilhaft wird der Köcher mittels des Verbindungselementes mit der Kontaktschicht lösbarverbunden und fixiert. Durch die sich von Verbindungselement und korrespondierender Aufnahme ausgebildete mechanische Verbindung wird ein stabiler mechanischer Halt und Sicherung durch das sich ausbildende Schlüssel-Schloss-Prinzip gewährleistet. In Ausführungsformen ist das Verbindungselement am distalen Ende angeordnet.

In weiteren Ausführungsformen ist die zum Verbindungselement korrespondierende Aufnahme am Köcherboden angeordnet.

In Ausführungsformen ist die Verbindung zwischen Verbindungselement am Schichtsystem und korrespondierender Aufnahme am Köcher lösbar ausgebildet. Vorteilhaft kann das erfindungsgemäße Schichtsystem somit leicht vom Köcher gelöst werden.

In Ausführungsformen weisen Stift, Verschlusssystem und/oder Absperrorgan ein Gewinde oder Getriebe wie bspw. ein Schneckengetriebe auf, welches mit einer korrespondierenden lösbar ausgebildeten Aufnahme am Köcher verbunden wird.

In Ausführungsformen ist das Verschlusssystem als mechanischer Verschluss wie bspw. ein Bajonettverschluss oder ein magnetischer Verschluss ausgebildet. Dabei weist der mechanische Verschluss zwei Teile auf, wobei der erste Teil dem Verbindungselement und der zweite Teil der am Köcher angeordneten korrespondierenden Aufnahme zuzuordnen ist.

In Ausführungsformen ist das Absperrorgan als Ventil ausgebildet.

In Ausführungsformen ist das Dichtungselement als Dichtungsring, Dichtungsfläche oder Dichtungslippe ausgebildet.

In bevorzugten Ausführungsformen wird eine Kombination von Stift oder Verschlusssystem oder Absperrorgan und dem Dichtungselement verwendet. Vorteilhaft wird dadurch der sich ausbildende Kraft- und oder Formschluss und Vakuum bzw. Unterdruck und damit der Halt erhöht.

In weiteren Ausführungsformen ist das Verbindungselement als Kordel ausgebildet.

In einer bevorzugten Ausführungsform weist die Kontaktschicht mindestens eine Haltevorrichtung auf, um in korrespondierende Gegenstücke an entsprechenden Positionen am Köcher einen zusätzlichen Form- und/oder Kraftschluss einzugehen. In Ausführungsformen ist die Haltevorrichtung am proximalen Ende angeordnet. In Ausführungsformen verläuft die mindestens eine Haltevorrichtung zumindest teilweise entlang, sozusagen umlaufend bzw. zirkulär, des proximalen Endes.

In weiteren Ausführungsformen ist die Haltevorrichtung als Erhebung ausgebildet. In weiteren Ausführungsformen ist die Haltevorrichtung als Noppe oder Dichtungselement wie beispielsweise Dichtungsring, Dichtungsfläche oder Dichtungslippe ausgebildet. Vorteilhaft wird durch die Haltevorrichtung ein zusätzlicher Form- und/oder Kraftschluss und somit ein besserer Halt und Sitz des erfindungsgemäßen Schichtsystems gewährleistet.

In einer bevorzugten Ausführungsform sind die stumpfabgewandte Fläche der Adhäsionsschicht und die stumpfzugewandte Fläche der Kontaktschicht über die sich so ausbildende Kontaktfläche zumindest teilweise miteinander verbunden.

In Ausführungsformen ist die genannte Verbindung nur am proximalen Ende des erfindungsgemäßen Schichtsystems ausgebildet. Dies bedeutet, dass die Verbindung durchgehend und somit entlang sozusagen umlaufend bzw. zirkulär des proximalen Endes verläuft. Dadurch entsteht ein Hohlraum, welcher jenem Volumen entspricht, welches sich zwischen Adhäsions- und Kontaktschicht im Bereich zwischen der Verbindung bildet. In Ausführungsformen ist die genannte Verbindung luftdicht ausgebildet, sodass vorteilhaft die Bildung eines Vakuums bzw. Unterdrucks im Hohlraum erleichtert wird. Dies erfolgt vorzugsweise mittels einer kraftschlüssigen oder formschlüssigen oder stoffschlüssigen Verbindung bzw. Kombinationen dieser Verbindungen. So sind bspw. Dichtungslippen, ineinandergreifende Dichtungen, Dichtungslippen mit mechanischer Spannung (die äußere Dichtungslippe übt eine Kraftwirkung auf die innere Dichtung aus und bildet so eine Kombination von Kraft- und Formschluss) oder bspw. ein Verkleben, Verschweißen oder eine integrierte Fertigung beider Schichten (mit verbundenen proximalen Enden) zu einem gemeinsamen Bauteil als Stoffschluss möglich. Vorzugsweise sind die beiden Schichten nur an den proximalen Enden, vollständig um den Stumpf umlaufend, stoffschlüssig miteinander verbunden.

In weiteren Ausführungsformen ist die genannte Verbindung gasdicht ausgebildet. Vorteilhaft erfolgt dadurch insbesondere am proximalen Ende eine Kraftwirkung aufgrund des Differenzdrucks, wodurch vorteilhaft eine effiziente Haltekraft erreicht wird. In Ausführungsformen erfolgt die genannte Verbindung durch ein Fügeverfahren, wobei das Fügeverfahren thermisch, mechanisch und/oder chemisch ist.

In weiteren Ausführungsformen ist die genannte Verbindung herstellungsbedingt durch das entsprechend gewählte Fügeverfahren als Wulst ausgebildet, welche zumindest teilweise entlang des proximalen Endes umlaufend bzw. zirkulär verläuft. Insbesondere ist die stumpfabgewandte Fläche der Adhäsionsschicht mit der der stumpfzugewandten Fläche der Kontaktschicht zumindest teilweise verklebt oder verschweißt. Als Wulst wird im Sinne der Erfindung eine verstärkte Umrandung verstanden.

In Ausführungsformen erfolgt die Herstellung von Adhäsions- und/oder Kontaktschicht des erfindungsgemäßen Schichtsystems durch additive Fertigungsverfahren oder durch gießen oder durch walzen. So kann das Gießen bei RTV-Silikonkautschuk oder das Walzen bei HTV-Silikonkautschuk angewendet werden.

In einer bevorzugten Ausführungsform besteht die Adhäsionsschicht und/oder die Kontaktschicht aus medizinischem Silikon, Polyethylen, Polyurethan oder einer Mischform dieser Materialien.

In Ausführungsformen handelt es sich bei medizinischem Silikon um ein vernetzendes Silikon, insbesondere Flüssigsilikon, RTV-Silikonkautschuk, HTV-Silikonkautschuk oder UV-vernetzbares Silikon.

In Ausführungsformen weisen die Adhäsionsschicht und/oder die Kontaktschichten dieselben Materialien auf.

In einer bevorzugten Ausführungsform ist zumindest teilweise eine Beschichtung zwischen der stumpfabgewandten Fläche der Adhäsionsschicht und der stumpfzugewandten Fläche der Kontaktschicht angeordnet.

In weiteren Ausführungsformen weisen sowohl die stumpfzugewandte Fläche der Adhäsionsschicht und die stumpfabgewandte Fläche der Kontaktschicht eine Beschichtung auf.

In weiteren Ausführungsformen weist nur die stumpfzugewandte Fläche der Adhäsionsschicht eine Beschichtung auf.

In weiteren Ausführungsformen weist nur die stumpfabgewandte Fläche der Adhäsionsschicht eine Beschichtung auf.

In weiteren Ausführungsformen weist nur die stumpfzugewandte Fläche der Kontaktschicht eine Beschichtung auf.

In weiteren Ausführungsformen weist nur die stumpfabgewandte Fläche der Kontaktschicht eine Beschichtung auf.

In Ausführungsformen ist die Beschichtung am distalen Ende ausgebildet.

In weiteren Ausführungsformen verläuft die Beschichtung zumindest teilweise umlaufend bzw. zirkulär entlang des proximalen Endes.

In Ausführungsformen ist die Beschichtung als Trennschicht bzw. Zwischenschicht ausgebildet. Insbesondere trennt die Beschichtung die Adhäsionsschicht und die Kontaktschicht voneinander. Vorteilhaft ist im Bereich der Beschichtung gezielt die Adhäsion außer Kraft gesetzt.

In weiteren Ausführungsformen ist die Beschichtung gleitfähig ausgebildet. Vorteilhaft wird dadurch das Auf- und Abrollen des erfindungsgemäßen Schichtsystems ermöglicht, da jene Bereiche mit Beschichtung keine Adhäsionswirkung aufweisen und somit die Rutschhemmung minimiert wird.

In einer bevorzugten Ausführungsform wird durch das erfindungsgemäße Schichtsystem der Bereich eines Körpergelenks des Trägers zum Teil oder gänzlich ausgespart. Dadurch bleiben die Körpergelenke so frei jeglicher Bedeckung und werden nicht von Materialschichten überragt. Vorteilhaft bietet dies für den Prothesenträger, welcher das erfindungsgemäße Schichtsystem trägt, mehr Komfort und Bewegungsfreiheit, insbesondere im Bereich der Gelenke. Vorteilhaft wird dadurch weiterhin die Schweißbildung oder Hautirritationen vermieden. Damit einhergehend können auch längere Tragezeiten realisiert werden. Im Gegensatz zu herkömmlichen Lösungen ist auch kein Umstülpen des erfindungsgemäßen Schichtsystems notwendig.

Bei dem Gelenk handelt es sich bspw. um das Kniegelenk oder Ellenbogengelenk.

Die Erfindung betrifft ferner eine Prothese, aufweisend ein erfindungsgemäßes Schichtsystem, einen Köcher und Prothesenbauteile, wobei das Schichtsystem am Stumpf ausbildbar angeordnet ist und der Köcher zwischen dem Schichtsystem und den Prothesenbauteilen angeordnet ist.

Weiterhin betrifft die Erfindung die Verwendung eines erfindungsgemäßen Schichtsystems und/oder eines erfindungsgemäßen Verfahrens für eine Prothese, wobei die Prothese als Bein- oder Armprothese ausgebildet ist.

Weiterhin betrifft die Erfindung die Verwendung eines erfindungsgemäßen Schichtsystems, wobei die Prothese als Bein- oder Armprothese ausgebildet ist.

Die Erfindung ist nicht auf die dargestellten und beschriebenen Ausführungsformen beschränkt, sondern umfasst auch alle im Sinne der Erfindung gleich wirkenden Ausführungsformen. Ferner ist die Erfindung auch nicht auf die speziell beschriebenen Merkmalskombinationen beschränkt, sondern kann auch durch jede beliebige andere Kombination von bestimmten Merkmalen aller insgesamt offenbarten Einzelmerkmale definiert sein, sofern sich die Einzelmerkmale nicht gegenseitig ausschließen, oder eine spezifische Kombination von Einzelmerkmalen nicht explizit ausgeschlossen ist.

Für die Realisierung der Erfindung ist es auch zweckmäßig, die vorbeschriebenen erfindungsgemäßen Ausgestaltungen, Ausführungsformen und Merkmale der Ansprüche in zweckmäßiger Anordnung miteinander zu kombinieren.

Weiter wird mit der Erfindung ein Verfahren zur Anbringung und/oder Entfernung einer Prothese an einen Stumpf umfassend als Schritte in folgender Reihenfolge: Aufrollen oder Abrollen des erfindungsgemäßen Schichtsystems auf einen Stumpf, Evakuierung oder Belüftung des Hohlraumes zwischen der Adhäsionsschicht und der Kontaktschicht durch ein Absperrorgan, Befestigung oder Entfernung eines Köchers und von Prothesenbauteilen an das erfindungsgemäße Schichtsystem durch ein Verbindungselement offenbart.

Im Sinne der Erfindung sind im Falle der Befestigung des Köchers an das erfindungsgemäße Schichtsystem auch gleichzeitig immer die Prothesenbauteile daran befestigt, da Köcher und Prothesenbauteile in der Regel miteinander verbunden sind. Bevor das Schichtsystem auf den Stumpf aufgerollt oder von diesem abgerollt wird, erfolgt das Öffnen des Absperrorgans, sodass Luft zwischen die Adhäsions- und Kontaktschicht in den Hohlraum strömen kann.

In Ausführungsformen wird das erfindungsgemäße Schichtsystem vor dem Aufrollen oder beim Abrollen auf links gedreht.

In Ausführungsformen entsteht bei der Evakuierung des Hohlraumes zwischen Adhäsions- und Kontaktschicht durch das Absperrorgan ein Vakuum bzw. Unterdruck.

Nachfolgend soll die Erfindung anhand eines Ausführungsbeispiels eingehender erläutert werden. Das Ausführungsbeispiel bezieht sich auf ein erfindungsgemäßes Schichtsystem und soll dabei die Erfindung beschreiben, ohne diese zu beschränken. Anhand von Zeichnungen wird die Erfindung näher erläutert. Dabei zeigen:
Fig. 1 - ein erfindungsgemäßes Schichtsystem in einer schematischen Schnittdarstellung,
Fig. 2 - ein erfindungsgemäßes Schichtsystem mit Haltevorrichtung und Köcher in einer schematischen Schnittdarstellung,
Fig. 3 - eine weitere Ausgestaltung des erfindungsgemäßen Schichtsystems in einer schematischen Schnittdarstellung.

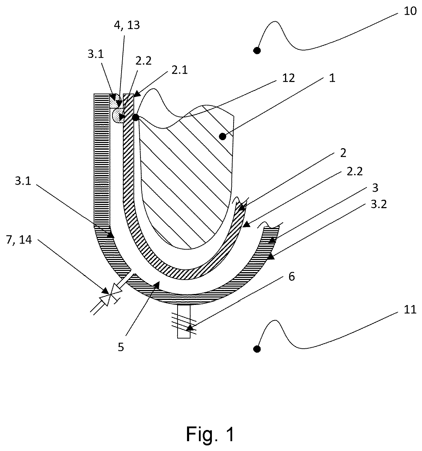

Figur 1 zeigt das erfindungsgemäße Schichtsystem in einer schematischen Schnittdarstellung, wobei die gezeigten Komponenten nicht maßstabsgetreu wiedergegeben sind. Das Schichtsystem weist ein proximales (10) und ein distales Ende (11) sowie eine Adhäsionsschicht (2) und eine Kontaktschicht (3) auf. Die Adhäsionsschicht (2) weist zwei Flächen (2.1, 2.2) auf, wobei die erste Fläche der Adhäsionsschicht (2.1) den Stumpf (1) bedeckt und sich zwischen Stumpf (1) und erster Fläche (2.1) eine Adhäsionskraft (12) durch die innewohnende Materialeigenschaft der Adhäsionsschicht (2) ausbildet.

Die Kontaktschicht (3) ist über der Adhäsionsschicht (2) angeordnet, bedeckt diese und weist zwei Flächen (3.1, 3.2) auf. Die Kontaktfläche (4) zwischen der zweiten Fläche der Adhäsionsschicht (2.2) und der ersten Fläche der Kontaktschicht (3.1) am proximalen Ende (10) des Schichtsystems ist durch Verschweißen und somit durch ein thermisches Fügeverfahren hergestellt. In diesem Zusammenhang bilden sich an der Kontaktfläche Wülste von der zweiten Fläche der Adhäsionsschicht (2.2) und der ersten Fläche der Kontaktschicht (3.1) aus, welche sich einander am proximalen Ende (10) berühren und umlaufend angeordnet sind.

Ferner ist die Kontaktfläche (4) über einen Kraft-Formschluss (13), welcher durch ein Absperrorgan (7) erreicht wird, luftdicht ausgebildet - durch das Absperrorgan (7) wird der Hohlraum (5) zwischen Adhäsionsschicht (2) und Kontaktschicht (3) evakuiert bzw. belüftet. Die zweite Fläche der Kontaktschicht (3.2) gewährleistet durch ein als Stift mit Gewinde ausgebildetes Verbindungselement (7) die Ausbildung eines weiteren Kraft- Formschlusses (14) durch eine Klemmkraft zu dem Köcher (nicht gezeigt). Der Köcher weist hierzu ein entsprechendes Gegenstück zur Aufnahme des Stiftes auf.

Die Adhäsionsschicht (2) und die Kontaktschicht (3) sind beide aus medizinischem Silikon hergestellt und weisen jeweils eine Schichtdicke von min. 2mm bis max. 6mm auf. Die Shorehärte ist dabei flächig individuell anpassbar.

Figur 2 zeigt das erfindungsgemäße Schichtsystem von Figur 1 mit Haltevorrichtung und einem zumindest teilweise angedeuteten Köcher in einer schematischen Schnittdarstellung, wobei die gezeigten Komponenten nicht maßstabsgetreu wiedergegeben sind. Die Haltevorrichtung ist aus mehreren, entlang der Kontaktfläche (3) zirkulär umlaufenden und sich erhebenden Dichtlippen ausgebildet. Die Haltevorrichtung dient dabei der zusätzlichen Sicherung des Schichtsystems an den Köcher.

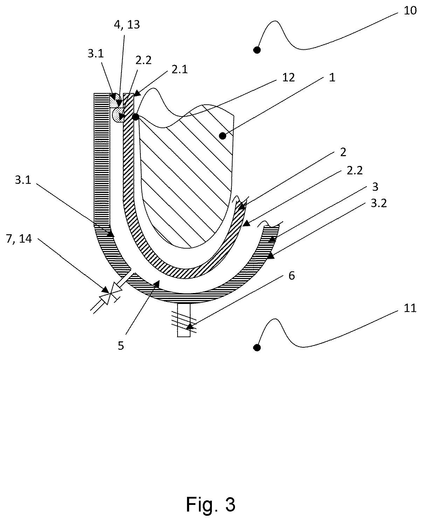

Figur 3 zeigt eine weitere Ausgestaltung des erfindungsgemäßen Schichtsystems in einer schematischen Schnittdarstellung, wobei die gezeigten Komponenten nicht maßstabsgetreu wiedergegeben sind. Die Zuordnung der Komponenten ist so wie in Figur 1 und 2 gezeigt. Die Kontaktfläche (4) zwischen der zweiten Fläche der Adhäsionsschicht (2.2) und der ersten Fläche der Kontaktschicht (3.1) am proximalen Ende (10) des Schichtsystems ist durch Verkleben hergestellt.

### Bezugszeichen

- 1: Stumpf
- 2: Adhäsionsschicht
- 2.1: erste Fläche der Adhäsionsschicht (stumpfzugewandt)
- 2.2: zweite Fläche der Adhäsionsschicht (stumpfabgewandt)
- 3: Kontaktschicht
- 3.1: erste Fläche der Kontaktschicht (stumpfzugewandt)
- 3.2: zweite Fläche der Kontaktschicht (stumpfabgewandt)
- 4: Kontaktfläche zwischen der zweiten ((stumpfabgewandt)) Fläche der Adhäsionsschicht und der ersten ((stumpfzugewandt)) Fläche der Kontaktschicht
- 5: Hohlraum
- 6: Verbindungselement
- 7: Absperrorgan
- 10: proximales Ende
- 11: distales Ende
- 12: Adhäsionskraft
- 13: Kraft- und/oder Formschluss zwischen Adhäsions- und Kontaktschicht
- 14: Kraft- und/oder Formschluss zwischen

## Patentansprüche

1. Schichtsystem zur Anbringung und/oder Entfernung eines Köchers an einen Stumpf (1), aufweisend ein proximales (10) und ein distales Ende (11), als Komponenten umfassend
- eine Adhäsionsschicht (2), aufweisend zwei Flächen (2.1, 2.2), wobei die stumpfzugewandte Fläche der Adhäsionsschicht (2.1) den Stumpf (1) beim Gebrauch zumindest teilweise bedeckt und einen Kontakt zum Stumpf (1) durch Ausbildung einer Adhäsionskraft (12) herstellt,
- eine Kontaktschicht (3), aufweisend zwei Flächen (3.1, 3.2), wobei die Kontaktschicht (3)
• über der stumpfabgewandten Fläche der Adhäsionsschicht (2.2) angeordnet ist, indem die stumpfzugewandte Fläche der Kontaktschicht (3.1) zumindest teilweise die zweite Fläche der Adhäsionsschicht (3.2) bedeckt,
• ausgebildet ist, über die stumpfabgewandte Fläche der Kontaktschicht (3.2) einen formschlüssigen Kontakt zu dem Köcher zu gewährleisten,
**dadurch gekennzeichnet, dass** das Schichtsystem
• ein Absperrorgan (7) zur Evakuierung und/oder Belüftung des zwischen Adhäsionsschicht (2) und Kontaktschicht (3) gebildeten Hohlraumes (5) aufweist, und
• am distalen Ende (11) ein Verbindungselement (6) zur lösbaren Befestigung des Schichtsystems an den Köcher aufweist,
• wobei die Kontaktfläche (4) zwischen der zweiten Fläche der Adhäsionsschicht (2.2) und der stumpfzugewandten Fläche der Kontaktschicht (3.1) am proximalen Ende (10) luftdicht ausgebildet ist.

2. Schichtsystem nach Anspruch 1, **dadurch gekennzeichnet, dass** das Absperrorgan (7) am distalen Ende (11) angeordnet ist.

3. Schichtsystem nach Anspruch 2, **dadurch gekennzeichnet, dass** das Absperrorgan ein Ventil ist.

4. Schichtsystem nach einem der Ansprüche 1 bis 3, **dadurch gekennzeichnet, dass** das Absperrorgan (7) und das Verbindungselement (6) als integriertes Bauteil ausgebildet ist.

5. Schichtsystem nach einem der Ansprüche 1 bis 4, **dadurch gekennzeichnet, dass** das Verbindungselement (6) als Stift und/oder Verschlusssystem und/oder Absperrorgan und/oder Dichtungselement ausgebildet ist.

6. Schichtsystem nach einem der Ansprüche 1 bis 5, **dadurch gekennzeichnet, dass** die Kontaktschicht (3) mindestens eine Haltevorrichtung aufweist, um in korrespondierende Gegenstücke an entsprechenden Positionen beim Köcher einen zusätzlichen Formschluss einzugehen.

7. Schichtsystem nach einem der Ansprüche 1 bis 6, **dadurch gekennzeichnet, dass** die die stumpfabgewandte Fläche der Adhäsionsschicht (2.2) und die stumpfzugewandte Fläche der Kontaktschicht (3.1) zumindest teilweise miteinander verbunden sind.

8. Schichtsystem nach einem der Ansprüche 1 bis 7, **dadurch gekennzeichnet, dass** die Adhäsionsschicht (2) und/oder die Kontaktschicht (3) aus Silikon, Polyethylen, Polyurethan oder einer Mischform dieser Materialien besteht.

9. Schichtsystem nach einem der Ansprüche 1 bis 8, **dadurch gekennzeichnet, dass** zumindest teilweise eine Beschichtung zwischen der stumpfabgewandten Fläche der Adhäsionsschicht (2.2) und der stumpfzugewandten Fläche der Kontaktschicht (3.1) angeordnet ist.

10. Schichtsystem nach einem der Ansprüche 1 bis 9, **dadurch gekennzeichnet, dass** der Bereich eines Körpergelenks zum Teil ausgespart wird.

11. Prothese, aufweisend ein Schichtsystem nach einem der Ansprüche 1 bis 9, einen Köcher und Prothesenbauteilen, wobei das Schichtsystem am Stumpf (1) ausbildbar angeordnet ist und der Köcher zwischen dem Schichtsystem und den Prothesenbauteilen angeordnet ist.

12. Verwendung eines Schichtsystems nach einem der Ansprüche 1 bis 9 für eine Prothese, wobei die Prothese als Bein- oder Armprothese ausgebildet ist.

## Claims

1. Layer system for attaching and/or removing a socket to a residual limb (1), having a proximal end (10) and a distal end (11), comprising as components
- an adhesion layer (2) having two surfaces (2.1, 2.2), wherein the limb-facing surface of the adhesion layer (2.1) at least partially covers the residual limb (1) during use and establishes contact with the residual limb (1) through the formation of
- an adhesion force (12), and a contact layer (3) having two surfaces (3.1, 3.2), wherein the contact layer (3)
∘ is arranged over the limb-opposite surface of the adhesion layer (2.2), such that the limb-facing surface of the contact layer (3.1) at least partially covers the second surface of the adhesion layer (2.2),
∘ is configured to provide positive-locking connection with the socket via the limb-opposite surface of the contact layer (3.2),
**characterized in that** the layer system
∘ comprises a closure element (7) for evacuating and/or ventilating a cavity (5) formed between the adhesion layer (2) and the contact layer (3), and
∘ comprises an attachment element (6) at the distal end (11) for releasably attaching the layer system to the socket,
∘ wherein the contact surface (4) between the second surface of the adhesion layer (2.2) and the limb-facing surface of the contact layer (3.1) is configured airtight at the proximal end (10).

2. Layer system according to claim 1, **characterized in that** the closure element (7) is arranged at the distal end (11).

3. Layer system according to claim 2, **characterized in that** the closure element is a valve.

4. Layer system according to any one of claims 1 to 3, **characterized in that** the closure element (7) and the attachment element (6) are formed as an integrated component.

5. Layer system according to any one of claims 1 to 4, **characterized in that** the attachment element (6) is designed as a pin and/or locking system and/or closure element and/or gasket element.

6. Layer system according to any one of claims 1 to 5, **characterized in that** the contact layer (3) comprises at least one retaining device configured to provide additional positive-locking engagement with corresponding counterparts at respective positions on the socket.

7. Layer system according to any one of claims 1 to 6, **characterized in that** the limb-opposite surface of the adhesion layer (2.2) and the limb-facing surface of the contact layer (3.1) are at least partially bonded to each other.

8. Layer system according to any one of claims 1 to 7, **characterized in that** the adhesion layer (2) and/or the contact layer (3) consist of silicone, polyethylene, polyurethane, or a mixture of these materials.

9. Layer system according to any one of claims 1 to 8, **characterized in that** a coating is arranged at least partially between the limb-opposite surface of the adhesion layer (2.2) and the limb-facing surface of the contact layer (3.1).

10. Layer system according to any one of claims 1 to 9, **characterized in that** the area of a body joint is partially omitted.

11. Prosthesis comprising a layer system according to any one of claims 1 to 9, a socket, and prosthetic components, wherein the layer system is arranged attachably on the residual limb (1), and the socket is arranged between the layer system and the prosthetic components.

12. Use of a layer system according to any one of claims 1 to 9 for a prosthesis, wherein the prosthesis is configured as a leg or arm prosthesis.

## Revendications

1. Système de couches pour la fixation et/ou le retrait d'un carquois sur un moignon (1), présentant une extrémité proximale (10) et une extrémité distale (11), comprenant comme composants
- une couche d'adhérence (2), présentant deux surfaces (2.1, 2.2), la surface de la couche d'adhérence (2.1) tournée vers le moignon recouvrant au moins partiellement le moignon (1) lors de l'utilisation et établissant un contact avec le moignon (1) par la formation d'une force d'adhérence (12),
- une couche de contact (3), présentant deux surfaces (3.1, 3.2), la couche de contact (3)
• étant disposée au-dessus de la surface de la couche d'adhérence (2.2) opposée au moignon, la surface de la couche de contact (3.1) tournée vers le moignon recouvrant au moins partiellement la deuxième surface de la couche d'adhérence (3.2),
• est conçue pour garantir un contact par complémentarité de forme avec le carquois par l'intermédiaire de la surface de la couche de contact (3.2) opposée au moignon, **caractérisé en ce que** le système de couches
• comporte un organe d'obturation (7) pour l'évacuation et/ou la ventilation de la cavité (5) formée entre la couche d'adhérence (2) et la couche de contact (3), et
• comporte à l'extrémité distale (11) un élément de liaison (6) pour la fixation amovible du système de couches au carquois,
• la surface de contact (4) entre la deuxième surface de la couche d'adhérence (2.2) et la surface de la couche de contact (3.1) tournée vers le moignon à l'extrémité proximale (10) étant étanche à l'air.

2. Système de couches selon la revendication 1, **caractérisé en ce que** l'organe d'arrêt (7) est disposé à l'extrémité distale (11).

3. Système de couches selon la revendication 2, **caractérisé en ce que** l'organe d'arrêt est une valve.

4. Système de couches selon l'une des revendications 1 à 3, **caractérisé en ce que** l'organe d'arrêt (7) et l'élément de liaison (6) sont conçus comme un composant intégré.

5. Système de couches selon l'une des revendications 1 à 4, **caractérisé en ce que** l'élément de liaison (6) est conçu comme une tige et/ou un système de verrouillage et/ou un organe d'arrêt et/ou un élément d'étanchéité.

6. Système de couches selon l'une des revendications 1 à 5, **caractérisé en ce que** la couche de contact (3) comporte au moins un dispositif de retenue afin de former une liaison supplémentaire par complémentarité de forme avec des contre-pièces correspondantes à des positions correspondantes sur le carquois.

7. Système de couches selon l'une des revendications 1 à 6, **caractérisé en ce que** la surface de la couche d'adhérence (2.2) opposée au moignon et la surface de la couche de contact (3.1) tournée vers le moignon sont au moins partiellement reliées entre elles.

8. Système de couches selon l'une des revendications 1 à 7, **caractérisé en ce que** la couche d'adhérence (2) et/ou la couche de contact (3) est constituée de silicone, de polyéthylène, de polyuréthane ou d'un mélange de ces matériaux.

9. Système de couches selon l'une des revendications 1 à 8, **caractérisé en ce qu'**un revêtement est disposé au moins partiellement entre la surface de la couche d'adhérence (2.2) opposée au moignon et la surface de la couche de contact (3.1) tournée vers le moignon.

10. Système de couches selon l'une des revendications 1 à 9, **caractérisé en ce que** la zone d'une articulation du corps est partiellement évidée.

11. Prothèse comprenant un système de couches selon l'une des revendications 1 à 9, un carquois et des composants de prothèse, le système de couches étant disposé de manière à pouvoir être formé sur le moignon (1) et le carquois étant disposé entre le système de couches et les composants de prothèse.

12. Utilisation d'un système de couches selon l'une des revendications 1 à 9 pour une prothèse, la prothèse étant conçue comme une prothèse de jambe ou de bras.
